# EUROPEAN PATENT SPECIFICATION

(11) **EP 3 318 234 B1**
(45) Date of publication and mention of the grant of the patent: **29.03.2023**
(21) Application number: 16817599.0
(22) Date of filing: 25.05.2016
(51) Int. Cl.: A61F 13/496, A61F 13/511, A61F 13/514, A61F 13/84

(54) **PULL-ON DISPOSABLE DIAPER**
WEGWERFWINDEL ZUM ANZIEHEN
COUCHE JETABLE À TIRER

(30) Priority: 30.06.2015 JP 2015131815
(43) Date of publication of application: 09.05.2018
(73) Proprietor: Unicharm Corporation, Ehime 799-0111 (JP)
(72) Inventor: NAGASE, Noriko, Kanonji-shi Kagawa 769-1602 (JP); YOSHIOKA, Toshiyasu, Kanonji-shi Kagawa 769-1602 (JP); FUKASAWA, Jun, Kanonji-shi Kagawa 769-1602 (JP)
(74) Representative: Dolleymores
(86) International application number: PCT/JP2016/065491
(87) International publication number: WO 2017/002490

(56) References cited:
- WO-A1-2008/001330
- WO-A1-2015/076047
- JP-A- H0 984 824
- JP-A- 2003 305 082
- JP-A- 2005 237 768
- JP-A- 2006 043 068
- JP-A- 2012 192 115
- JP-A- 2012 192 115
- US-A1- 2005 155 892
- US-A1- 2006 241 559
- US-A1- 2010 152 694

## Description

### Technical Field

The present invention relates to a pull-on disposable diaper.

### Background Art

Conventionally, pull-on disposable diapers have been widely used for children and the like. This pull-on disposable diaper includes an absorbent main body provided with an absorbent body for absorbing bodily wastes, a back waist portion positioned on one end side of the absorbent main body, and a front waist portion positioned on the other end side of the absorbent main body. When such a disposable diaper is to be put on a newborn infant, it is required that the newborn infant's navel does not come into contact with the disposable diaper since the navel is moist. For example, Patent Document 1 discloses a diaper in which a cutout having a downward protruding shape is provided in a part of the upper end edge of the front waist portion (the front), thereby restraining the front waist portion from coming into contact with a newborn infant's navel, etc., when the diaper is put on.

### Citation List

### Patent Document

Patent Document 1: Japanese Patent Application Publication No. 2012-192115A
WO 2008/001330 A1 discloses a diaper including at least one serviceable indicium proximate a front waist region that facilitates fitting of the diaper. The serviceable indicium has a curvature for alignment with an anatomical feature of the wearer.
US 2006/241559 A1 relates to a diaper having a front portion with at least one line of weakness adapted to be torn to provide an optional umbilical feature.

### Summary of Invention

### Technical Problem

Commonly, the front side (stomach side) and the rear side (back side) of pull-on disposable diapers have almost the same shape, color, etc., thus making it difficult to distinguish between the front and rear. Diapers for newborn infants in particular are small, and a cutout provided in the upper end edge of the front waist portion (front panel) is not prominent, and thus problems may occur such as the cutout not being recognized and the diaper 1 being put on backwards.

The present disclosure has been achieved in view of the above-described problems, and an object is to make a cutout easier to recognize in a pull-on disposable diaper that has a cutout on the front side.

### Solution to Problem

In order to achieve an object described above, the present invention provides the pull-on disposable diaper of independent claim 1. The dependent claims specify preferred but optional features.

Other features of the present invention will become apparent from the description of the present specification and the accompanying drawings.

### Advantageous Effects of Invention

According to the present invention, it is possible to make a cutout easier to recognize in a pull-on disposable diaper that has a cutout on the front side.

### Brief Description of the Drawings

[Fig. 1] FIG. 1 is a schematic perspective view of a pull-on disposable diaper 1 according to a first embodiment.
[Fig. 2] FIG. 2 is a plan view of a diaper 1 in an unfolded state.
[Fig. 3] FIG. 3 is a schematic cross-sectional view taken along line A-A of FIG. 2.
[Fig. 4] FIG. 4A is a diagram illustrating a cutout 45. FIG. 4B is a diagram illustrating a variation of the cutout 45.
[Fig. 5] FIG. 5A and FIG. 5B are diagrams illustrating an example of the case where a separate sheet member 50 provided on the front side has a different color.
[Fig. 6] FIG. 6 is a diagram illustrating an example of the case where a portion on the front side of a back-sheet member 15 of an absorbent main body 10 has a different color.
[Fig. 7] FIG. 7 is a diagram illustrating an example of the case where a portion on the back side of a top-sheet member 13 of the absorbent main body 10 has a different color.
[Fig. 8] FIG. 8 is a diagram illustrating an example of the case where a partial region on the non-skin side of a front waist portion 40 has a different color.
[Fig. 9] FIG. 9 is a diagram illustrating an example of the case where a partial region on the skin side of a back waist portion 30 has a different color.
[Fig. 10] FIG. 10 is a schematic cross-sectional view of the diaper 1 for illustrating an example of the case where the configuration of a non-skin side sheet 32 has been changed.
[Fig. 11] FIG. 11 is a diagram illustrating an example of the case where partial regions of elastic strings 43a and 43b have a different color.

### Description of Embodiments

At least the following matters will become apparent from the description of the present specification and the accompanying drawings.

A pull-on disposable diaper having a vertical direction and a lateral direction intersecting the vertical direction, the pull-on disposable diaper including: an absorbent main body provided with an absorbent body that absorbs bodily wastes; a back waist portion located on one end side of the absorbent main body; and a front waist portion located on another end side of the absorbent main body, the front waist portion including a cutout, the cutout being located in an upper end portion of the front waist portion in the vertical direction and in a central portion of the front waist portion in the lateral direction, and in a view from a front side, at least a partial region of an edge portion including an outer edge of the cutout in the front waist portion and at least a partial region of the back waist portion having mutually different colors separated at the outer edge of the cutout.

According to this pull-on disposable diaper, it can be seen that the edge portion of the front waist portion and the back waist portion have mutually different colors separated at the outer edge of the cutout. Accordingly, the edge portion is more prominent, and the user can more easily recognize the position and shape of the cutout. Also, the existence of the cutout is more easily recognized, and therefore it is easier to distinguish between the front and the back of the diaper.

In this pull-on disposable diaper, a sheet member overlapped with at least a part of the edge portion is overlaid in the front waist portion, and in a view from the front side, an overlap region of the sheet member and the edge portion and at least a partial region of the back waist portion have mutually different colors separated at the outer edge of the cutout.

According to this pull-on disposable diaper, it can be seen that the back waist portion and the sheet member provided in the portion overlapped with the edge portion of the front waist portion have mutually different colors separated at the outer edge of the cutout, thus making it easier to recognize the cutout. Also, by changing the size, arrangement, color, or the like of the sheet member, it is possible to allow the user to more easily recognize the cutout according to differences in the type, shape, size, and the like of the pull-on disposable diaper.

In this pull-on disposable diaper, it is desirable that the sheet member is a design sheet provided on the front waist portion, and the design sheet has a region overlapped with the edge portion, and a color is given to at least a part of the region overlapped with the edge portion.

According to this pull-on disposable diaper, by providing a design that makes the region overlapped with the edge portion more prominent, the position and the shape of the cutout are recognized more easily, and it is possible to enhance the design of the pull-on disposable diaper. Also, by overlaying the design sheet, the number of sheets in the front waist portion increases, thus making it possible to increase the strength of the portion where the design sheet is overlaid.

In this pull-on disposable diaper, it is desirable that the absorbent main body has a top sheet that covers the absorbent body from a skin side, and a back sheet that covers the absorbent body from a non-skin side, the sheet member is the back sheet, and the back sheet has a region overlapped with the edge portion, and a color is given to at least a part of the region overlapped with the edge portion.

According to this pull-on disposable diaper, by merely giving a color to a portion of the back sheet of the absorbent main body, the position and the shape of the cutout can be recognized easily. Also, there is no need to provide a separate sheet member that is different from the back sheet, thus facilitating restraining an increase in the complexity of the manufacturing process and a rise in manufacturing cost.

In this pull-on disposable diaper, it is desirable that a sheet member is overlaid in the back waist portion, the sheet member being overlapped with at least a part of a position facing the cutout, and in a view from a front side, the edge portion and an overlap region of the position facing the cutout and the sheet member have mutually different colors separated at the outer edge of the cutout.

According to this pull-on disposable diaper, it can be seen that the edge portion of the cutout provided in the front waist portion and the sheet member provided at the position facing the cutout in the back waist portion have mutually different colors separated at the outer edge of the cutout, thus making it easier to recognize the cutout. Also, by changing the size, arrangement, color, or the like of the sheet member, it is possible to allow the user to more easily recognize the cutout according to differences in the type, shape, size, and the like of the pull-on disposable diaper.

In this pull-on disposable diaper, it is desirable that the absorbent main body has a top sheet that covers the absorbent body from a skin side, and a back sheet that covers the absorbent body from a non-skin side, the sheet member is the top sheet, and the back sheet has a region where the sheet member and the position facing the cutout are overlapped, and a color is given to at least a part of the region where the sheet member and the position facing the cutout are overlapped.

According to this pull-on disposable diaper, by merely giving a color to a portion of the top sheet of the absorbent main body, the position and the shape of the cutout can be recognized easily. Also, there is no need to provide a separate sheet member that is different from the top sheet, thus facilitating restraining an increase in the complexity of the manufacturing process and a rise in manufacturing cost.

In this pull-on disposable diaper, it is desirable that a color is given to at least a partial region of the edge portion on a non-skin side surface of the front waist portion.

According to this pull-on disposable diaper, by giving a color to a portion of the surface design of the diaper, for example, it is possible to facilitate recognition of the cutout portion while also improving the design of the diaper. Also, if members having mutually different colors are used for a member (nonwoven fabric) configuring the non-skin side of the front waist portion and a member (nonwoven fabric) configuring the back waist portion, it is possible to omit a step of printing or the like.

In this pull-on disposable diaper, it is desirable that a skin-side surface of the back waist portion has a position facing the cutout, and a color is given to at least a partial region of the position facing the cutout.

According to this pull-on disposable diaper, by giving a color to a portion of the skin-side surface of the back waist portion, the position and the shape of the cutout can be recognized easily. Also, if members having mutually different colors are used for a member (nonwoven fabric) configuring the skin side of the back waist portion and a member (nonwoven fabric) configuring the front waist portion, it is possible to omit a step of printing or the like.

In this pull-on disposable diaper, it is desirable that the back waist portion has a skin-side sheet and a non-skin side sheet overlaid in the thickness direction, the non-skin side sheet has a protruding portion protruding upward from an upper end portion of the skin-side sheet, and the protruding portion is folded back on a skin side, and a folded-back portion of the non-skin side sheet has a position facing the cutout, and a color is given to at least a partial region of the position facing the cutout.

According to this pull-on disposable diaper, by giving a color to a part of the folded-back portion of the non-skin side sheet, the position and the shape of the cutout can be recognized easily. Also, the folded-back non-skin side sheet restrains the wearer's skin from coming into direct contact with the upper end of the skin-side sheet, which can provide more favorable texture for the diaper.

In this pull-on disposable diaper, it is desirable that the front waist portion is provided with an elastic member that gives stretchability in the lateral direction, the elastic member being overlapped with at least a part of the edge portion, and the elastic member has a region overlapped with the edge portion, and a color is given to at least a part of the region overlapped with the edge portion.

According to this pull-on disposable diaper, by giving a color to a part of the elastic member (e.g., an elastic string) provided in the vicinity of the upper end of the front waist portion, the position and the shape of the cutout can be recognized easily. Also, if an elastic member that has been given a color or the like in advance is used, there is no need for color provision or the like in the manufacturing process, thus making manufacturing of the diaper simpler, and also making it possible to restrain a rise in manufacturing cost.

In this pull-on disposable diaper, a color difference between a color in at least a partial region of the edge portion including the outer edge of the cutout in the front waist portion and a color in at least a partial region of the back waist portion is greater than or equal to 1.5, the color difference being calculated based on an L*a*b* color coordinate system.

According to this pull-on disposable diaper, the difference between the colors of the two regions can be clearly recognized with the unaided eye, thus making it possible for the user to recognize the position and the shape of the cutout at a glance, and also restraining mistakes regarding the front and back of the diaper, for example.

### ===First Embodiment===

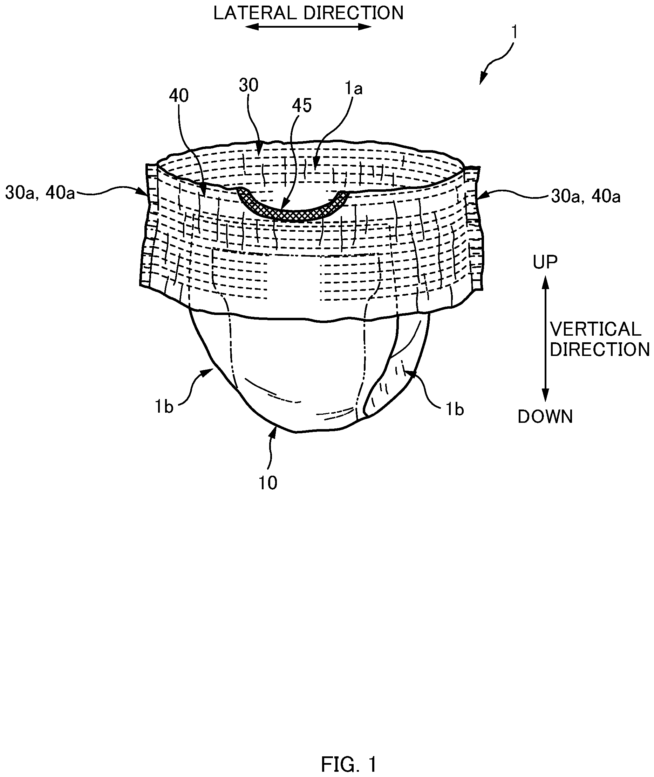

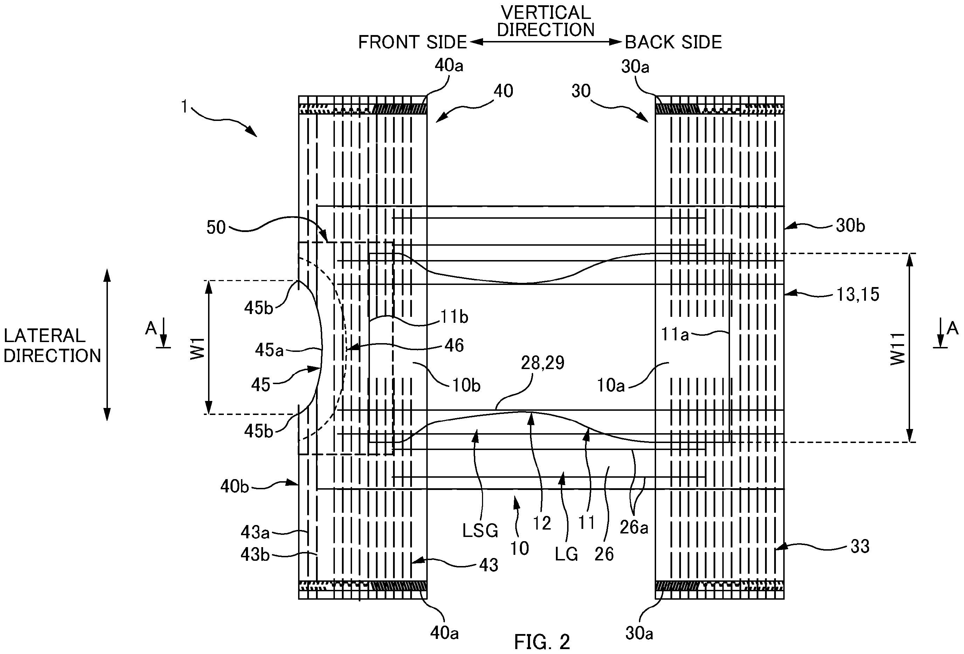

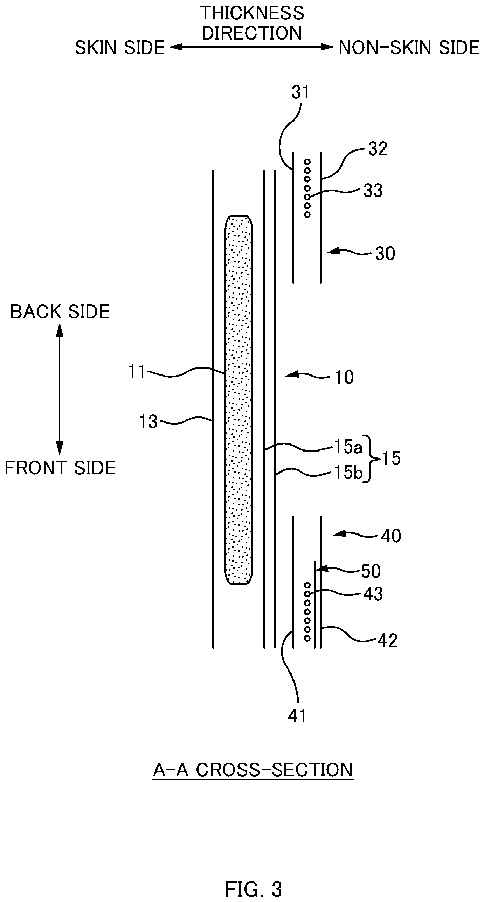

FIG. 1 is a schematic perspective view of a pull-on disposable diaper 1 (hereinafter, simply referred to as the diaper 1) according to a first embodiment. FIG. 2 is a plan view of the diaper 1 in an unfolded state. FIG. 3 is a schematic cross-sectional view taken along line A-A of FIG. 2.

This diaper 1 is a pull-on disposable diaper that is worn mainly by a newborn infant, and has a vertical direction and a lateral direction intersecting each other in the pull-on (pant-shaped) state in FIG. 1. Further, the lateral direction in its unfolded state in FIG. 2 is synonymous with the lateral direction in the pull-on state in FIG. 1. The vertical direction in the unfolded state is along the vertical direction in the pull-on state, but these two are slightly different in sense. For this reason, in the following descriptions, there are cases where the vertical direction in FIG. 2 is referred to as the "vertical direction in the unfolded state" or the like, as necessary, in order to distinguish it from the vertical direction in the pull-on state. Also, as illustrated in FIG. 3, with respect to the thickness direction, the side coming into contact with a wearer will be referred to as the "skin side", and the side opposite thereto will be referred to as the "non-skin side".

Further, the diaper 1 according to an embodiment of the present disclosure is a so-called three-piece type in diaper, and has three parts. Specifically, this diaper 1 includes an absorbent main body 10 configured to be placed against the wearer's crotch portion and absorb bodily wastes such as urine, as the first part; has a back waist portion 30 that covers the wearer's back portion, as the second part; and has a front waist portion 40 that covers the wearer's front portion, as the third part.

In the unfolded state in FIG. 2, the back waist portion 30 and the front waist portion 40 are arranged parallel to each other with a space therebetween in the vertical direction (vertical direction in the unfolded state), and the absorbent main body 10 spans this space. The back waist portion 30 is fixed to one end portion 10a of the absorbent main body 10, the front waist portion 40 is fixed to the other end portion 10b, and the external shape is a substantially H-shaped in a plan view. In other words, the back waist portion 30 is located on one end side of the absorbent main body 10, and the front waist portion 40 is located on the other end side of the absorbent main body 10.

The absorbent main body 10 in this state is then folded into two at a folding position that is a position in the center of the diaper 1 in the lengthwise direction (i.e., vertical direction) of the absorbent main body 10 (the center between the one end and the other end in the vertical direction of the diaper 1 in the unfolded state) . When the back waist portion 30 and the front waist portion 40, which face each other in this bi-fold state, are bonded/coupled in the lateral direction, these waist portions form a ring shape. Specifically, the diaper 1 is formed by coupling (bonding) both back-side edge portions 30a in the lateral direction of the back waist portion 30 to both front-side edge portions 40a in the lateral direction of the front waist portion 40, respectively. Accordingly, this results in the diaper 1 in a state when it is worn in which a waist opening 1a and a pair of leg openings 1b illustrated in FIG. 1 are formed. Note that in an embodiment of the present disclosure, the two back-side edge portions 30a and the two front-side edge portions 40a are respectively bonded in a state in which the position in the vertical direction of an upper end 40b of the front waist portion 40 in the vertical direction is matched with the position in the vertical direction of an upper end 30b of the back waist portion 30 in the vertical direction. In other words, the upper ends of the two back-side edge portions 30a and the upper ends of the two front-side edge portions 40a are respectively bonded.

The following describes three parts, which are the absorbent main body 10, the front waist portion 40, and the back waist portion 30, in this order.

The absorbent main body 10 corresponds to the wearer's crotch (inseam region) and is for absorbing bodily wastes such as urine. As illustrated in FIG. 2, the absorbent main body 10 includes a rectangular shape in a plan view, and is provided such that the lengthwise direction (long-side direction) is along the vertical direction in the unfolded state. Also, as illustrated in FIG. 3, the absorbent main body 10 includes an absorbent body 11, a top-sheet member 13 that covers the absorbent body 11 from the skin side, and a back-sheet member 15 that covers the absorbent body 11 from the non-skin side and forms the exterior of the absorbent main body 10.

The absorbent body 11 is a member (absorbent core) obtained by overlapping layers of a liquid-absorbent material, and can absorb bodily wastes such as urine. The absorbent body 11 has pulp (pulp fibers) and an absorbent polymer (SAP) . Note that the absorbent body 11 according to an embodiment of the present disclosure is substantially hourglass-shaped in a plan view. Specifically, the absorbent body 11 in the unfolded state includes a recessed portion 12 that is recessed laterally inward in the central portion in the vertical direction of the absorbent body 11, and the width of the absorbent body 11 is narrowest in the recessed portion 12. Note that the position of the recessed portion 12 in the vertical direction is to the front waist portion 40 side from the center in FIG. 2, but there are also cases where the position of the recessed portion 12 in the vertical direction is to the back waist portion 30 side depending on the size of the diaper 1. On the other hand, the recessed portion 12 is not provided in the end portions in the vertical direction of the absorbent body 11 (back-side upper end 11a and front-side upper end 11b of the absorbent body 11), and the width of the absorbent body 11 is largest in these end portions (back-side upper end 11a and front-side upper end 11b of the absorbent body 11) . In an embodiment of the present disclosure, a maximum width W11 of the absorbent body 11 is substantially 120 mm. Also, the absorbent body 11 may be covered with a core wrap (not illustrated) made of a liquid-permeable sheet such as tissue paper or nonwoven fabric.

The top-sheet member 13 is a sheet member made of a liquid-permeable nonwoven fabric or the like that has a planar size larger than that of the absorbent body 11, for example. The back-sheet member 15 is a sheet member that has a planar size larger than that of the absorbent body 11, for example, and one example is a sheet having a two-layer structure constituted by adhering together a liquid-impermeable leak-proof sheet 15a made of polyethylene, polypropylene, or the like and an exterior sheet 15b made of nonwoven fabric or the like. The absorbent body 11 is sandwiched between the back-sheet member 15 and the top-sheet member 13, and then the portions of the back-sheet member 15 and the top-sheet member 13 that lie outside from the four sides of the absorbent body 11 adhere to each other in a frame-like manner, thereby forming the absorbent main body 10. Note that a configuration may be such that the back-sheet member 15 is constituted by only the leak-proof sheet 15a without the exterior sheet 15b.

Further, a leg gather LG (leg stretching portion) that stretches and contract along the vertical direction is provided in portions respectively located on the sides in the lateral direction of the absorbent main body 10, namely each of side flaps 26 in a pair. The leg gathers LG are formed from nonwoven fabric, and include elastic members (specifically, elastic strings, which are referred to as LG elastic strings 26a for convenience) that stretch and contract along the vertical direction. The LG elastic strings 26a give stretchability to the side flaps 26, thereby configuring the leg gathers LG.

Further, a pair of leg side gathers LSG (barrier cuffs) are provided on the inner side with respect to the leg gathers LG (side flaps 26) in the lateral direction of the absorbent main body 10. The leg side gathers LSG are provided on the skin side of the absorbent main body 10 and serve to restrain liquid leakage through gaps around the legs. The leg side gathers LSG are formed from nonwoven fabric. The leg side gathers LSG each include a top part 28 at the inner end portion of the leg side gather LSG in the lateral direction of the absorbent main body 10, and the top part 28 and the nonwoven fabric on the outer side with respect to the top part 28 are configured to stand up. Further, the top parts 28 of the leg side gathers LSG are provided with elastic members (specifically, elastic strings, which are referred to as LSG elastic strings 29 for convenience) that stretch and contract along the vertical direction.

The front waist portion 40 is a sheet-like member that covers the wearer's abdomen. This front waist portion 40 is a sheet that is made of a nonwoven fabric or the like and has a rectangular shape in plan view, and is provided such that the lengthwise direction (long-side direction) is along the lateral direction. As illustrated in FIG. 3, the front waist portion 40 is formed by a skin-side sheet 41 and a non-skin side sheet 42 being layered from the skin side in the thickness direction and then bonded together. The front waist portion 40 is layered on the other end portion 10b of the absorbent main body 10 and bonded and fixed thereto. The portion of the front waist portion 40 that is placed on the absorbent main body 10 is provided in the central portion of the front waist portion 40 in the lateral direction.

Further, a separate sheet member 50, which is a sheet member that is different from the skin-side sheet 41 and the non-skin side sheet 42, is provided overlaid in the thickness direction in the front waist portion 40. In FIG. 3, the separate sheet member 50 is provided sandwiched between the skin-side sheet 41 and the non-skin side sheet 42, but the separate sheet member 50 may be provided on the non-skin side relative to the non-skin side sheet 42 (i.e., the most exterior of the front waist portion 40), or may be provided on the skin side relative to the skin-side sheet 41. In other words, the separate sheet member 50 is a sheet that is provided on the non-skin side relative to the absorbent main body 10 in the front waist portion 40. The separate sheet member 50 in an embodiment of the present disclosure is configured by a film, nonwoven fabric, or the like, and is a design sheet with a predetermined color or image provided on its surface. Provision of such a separate sheet member 50 can enhance the design of the diaper 1. Further, it is possible to increase strength in the region of the front waist portion 40 in which the separate sheet member 50 is overlaid. Details of the size, arrangement, etc. of the separate sheet member 50 in an embodiment of the present disclosure will be described later.

A cutout 45 is provided in a location that is in the central portion in the lateral direction of the front waist portion 40 and is at the upper end 40b in the vertical direction. This cutout 45 is for restraining the wearer's navel, a clip attached to the navel, or the like from coming into contact with the diaper 1.

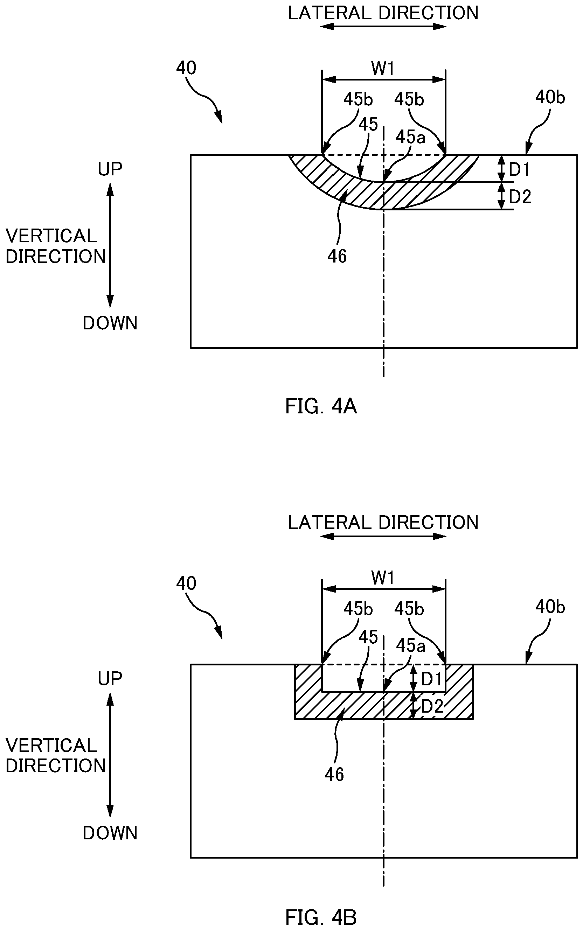

FIG. 4A is a diagram illustrating the cutout 45. The cutout 45 according to an embodiment of the present disclosure is formed in an arc-shape that curves downward in the vertical direction as illustrated in FIG. 4Awith the diaper 1 in the pull-on state. A depth D1 in the vertical direction of the cutout 45 is represented by the distance between the upper end 40b in the vertical direction of the front waist portion 40 and a lower end 45a in the vertical direction of the cutout 45. In other words, the depth D1 is the maximum length in the vertical direction of the cutout 45. In an embodiment of the present disclosure, the depth D1 is, for example, substantially 15 mm. The width W1 in the lateral direction of the cutout 45 is represented by the distance between two side ends 45b, which are points of intersection between the upper end 40b in the vertical direction of the front waist portion 40 and the cutout 45. In other words, the width W1 is the maximum length in the lateral direction of the cutout 45. In an embodiment of the present disclosure, the width W1 is, for example, substantially 85 mm. This width W1 is smaller than the previously-mentioned maximum width W11 (120 mm) of the absorbent body 11, and the side ends 45b of the cutout 45 are both located inward in the lateral direction relative to the side ends 15 of the widest portion of the absorbent body 11 (see FIG. 2).

Note that in an actual product, the width W1 of the cutout 45 when the product is viewed in a flat state is substantially 20 mm to 40 mm because the front waist portion 40 contracts in the lateral direction due to the action of elastic members such as front elastic strings 43, which will be described later. It should be noted that there are also cases where this width is greater or smaller depending on the degree of contraction of the elastic members. The lengths of the above-described depth D1 and width W1 are lengths measured in the state where the front waist portion 40 is spread out flat when contractive force is not exhibited by various elastic members such as the front elastic strings 43.

The front waist portion 40 includes an edge portion 46, which is a region that includes an outer edge of the cutout 45 and has a predetermined width. In the region illustrated by hatching in FIG. 4A, the edge portion 46 is provided in a range defined by a distance D2 from the outer edge of the cutout 45. In other words, the edge portion 46 is a region with the width D2 extending along the shape of the cutout 45. In an embodiment of the present disclosure, the width D2 of the edge portion 46 is a length equivalent to the depth D1 of the cutout 45 (D1=D2). It should be noted that the minimum value of the width D2 is 10 mm, and when the depth D1 is less than 10 mm, there are cases where the width D2 exceeds the depth D1 (D1<D2).

Further, the cutout 45 is not necessarily required to be arc-shaped, and the shape is not limited. FIG. 4B is a diagram illustrating a variation of the cutout 45. The cutout 45 of this variation is rectangular with the depth D1 and the width W1 as illustrated in FIG. 4B. In this case as well, similarly to the case in FIG. 4A, the edge portion 46 is provided in a region having the width D2 and including the outer edge of the rectangular cutout 45. Further, besides this rectangular shape, for example, another shape such as a triangle or a trapezoid that protrudes downward in the vertical direction may be used.

Returning to FIG. 2, multiple elastic members (specifically, elastic strings, which will be referred to as front elastic strings 43 for convenience) that stretch and contract along the lateral direction are arranged side-by-side in the vertical direction in the front waist portion 40. The front elastic strings 43 are bonded and fixed with an adhesive or the like to the front waist portion 40 in the state of being extended in the lateral direction. Accordingly, the front waist portion 40 is given stretchability in the lateral direction, and the waist opening 1a of the diaper 1 is given stretchability.

Among the plurality of the front elastic strings 43 that are provided, a predetermined number of elastic strings 43 including an elastic string 43a arranged nearest to the upper end 40b in the vertical direction of the front waist portion 40 have a portion that is overlapped with the edge portion 46 of the cutout 45 in a central portion in the lateral direction. In FIG. 2, two elastic strings 43a and 43b are arranged side-by-side in the vertical direction (the vertical direction in the unfolded state) so as to be overlapped with the edge portion 46. In other words, these two elastic strings 43a and 43b are both provided higher in the vertical direction than the lower end 45a of the cutout 45.

The back waist portion 30 is a sheet-like member that covers the wearer's back. This back waist portion 30 is a sheet that is made of a nonwoven fabric or the like and has a rectangular shape in plan view, and is provided such that its lengthwise direction (long-side direction) is along the lateral direction. As illustrated in FIG. 3, the back waist portion 30 is formed by a skin-side sheet 31 and a non-skin side sheet 32 being layered from the skin side in the thickness direction, and then bonded together. The back waist portion 30 is placed on the one end portion 10a of the absorbent main body 10 and bonded and fixed thereto. The portion of the back waist portion 30 that is placed on the absorbent main body 10 is provided in the central portion in the lateral direction of the back waist portion 30. Also, similarly to the front waist portion 40, a separate sheet member 50 may be provided to the back waist portion 30.

Note that, unlike the front waist portion 40, the back waist portion 30 is not provided with a cutout 45. The reason for this is that the objective of the cutout 45 is to restrain the wearer's navel, a clip attached to the navel, or the like from coming into contact with the diaper 1.

Substantially similarly to the front waist portion 40, multiple elastic members (specifically, elastic strings, which will be referred to as back elastic strings 33 for convenience) that stretch and contract along the lateral direction are arranged side-by-side in the vertical direction in the back waist portion 30 (see FIG. 2). The back elastic strings 33 are bonded and fixed with an adhesive or the like to the back waist portion 30 in the state of being extended in the lateral direction. This provides the back waist portion 30 with stretchability in the lateral direction, and provides the waist opening 1a of the diaper 1 with stretchability.

### <Identifiability of cutout 45>

Although the front waist portion 40 of the diaper 1 is provided with the above-described cutout 45, the cutout 45 has a small size in the first place, and also shrinks in the lateral direction due to stretchability provided by the front elastic strings 43, and therefore is difficult to identify in the normal state. In view of this, in the diaper 1 of an embodiment of the present disclosure, by improving identifiability in the cutout 45, the user (wearer) can identify the cutout 45 at a glance.

Specifically, when the diaper 1 is viewed from the front side, at least a partial region of the edge portion 46 including the outer edge of the cutout 45 provided in the front waist portion 40 and at least a partial region of the back waist portion 30 have mutually different colors separated at the outer edge of the cutout 45, and therefore the outer edge of the cutout 45 is more prominent. Accordingly, the user (wearer) can recognize the position and the shape of the cutout 45, and can correctly distinguish between the front and back directions of the diaper 1. Here, the phrase "when the diaper 1 is viewed from the front side" refers to viewing the diaper 1 from the front waist portion 40 side (i.e., the front side) in the state where the front waist portion 40 and the back waist portion 30 of the pull-on type of diaper 1 are overlapped in a flat state (product is laid flat) . For example, this is a case of viewing the diaper 1 from above in the state where the diaper 1 is placed on a flat surface such as a desk with the front side up, or a case of viewing the diaper 1 from the front waist portion 40 side in the state where the waist portions 40 and 30 are overlapped and stretched so as to be spread outward in the lateral direction.

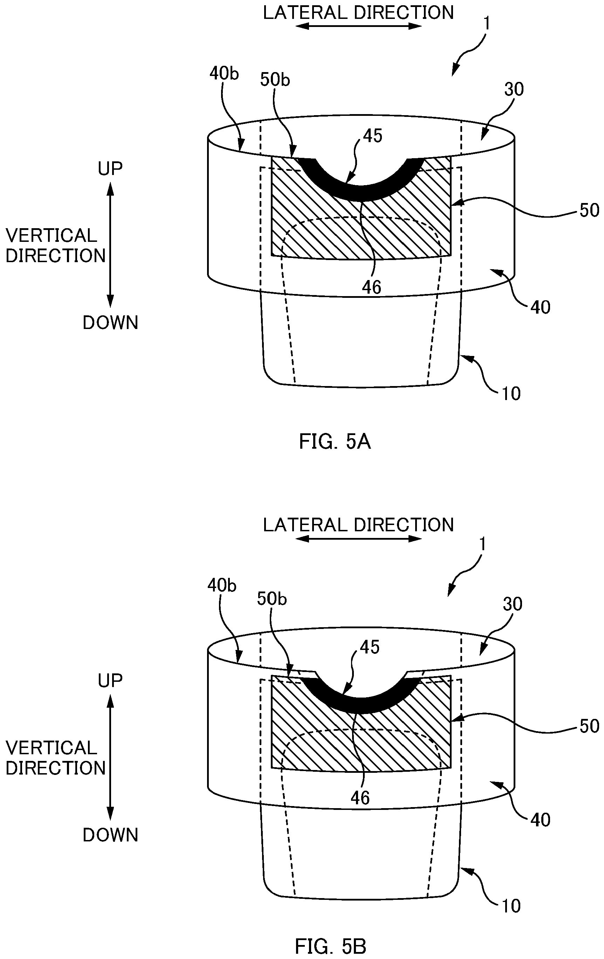

In the first embodiment, the color of a predetermined portion of a sheet member that is overlaid in the thickness direction in at least one of the front waist portion 40 and the back waist portion 30 is set to a different color from the color of the nonwoven fabric that configures the front waist portion 40 and the back waist portion 30, and thus the outer edge of the cutout 45 is more prominent. FIGS. 5A and 5B are diagrams illustrating an example of the case where the separate sheet member 50 provided on the front side has a different color.

In FIG. 5A, the above-described separate sheet member 50 is used as the sheet member overlaid in the waist portion. The width in the lateral direction of the separate sheet member 50 is larger than the width W1 of the cutout 45, and the length in the vertical direction of the separate sheet member 50 is set larger than the depth D1 of the cutout 45. Also, the upper end 50b in the vertical direction of the separate sheet member 50 is arranged so as to be overlapped with the upper end 40b in the vertical direction of the front waist portion 40. Accordingly, the separate sheet member 50 is overlapped with the entire region of the edge portion 46 in the thickness direction. Note that the cutout 45 is formed by overlaying the skin-side sheet 41 and the non-skin side sheet 42 of the front waist portion 40 with the separate sheet member 50, and then using a cutter or the like to cut these sheet members in an integrated manner.

In FIG. 5A, the region where the separate sheet member 50 and the edge portion 46 are overlapped (overlap region) is illustrated in solid black. In an embodiment of the present disclosure, in at least a part of this overlap region, the color of the separate sheet member 50 is different from the color of the nonwoven fabric that configures the back waist portion 30. For example, in FIG. 5A, assuming that the entirety of the region illustrated by the hatched portion of the separate sheet member 50 has been given a color, the edge portion 46 illustrated in solid black has also been given a color. Accordingly, when the diaper 1 is viewed from the skin side, the edge portion 46 of the front waist portion 40 and the back waist portion 30 have mutually different colors separated at the outer edge of the cutout 45, and the edge portion 46 is more prominent, thus making it easier for the user to recognize the cutout 45.

Note that the state where two regions "have mutually different colors" refers to a state where the color difference between the two regions for comparison can be perceived with the unaided eye. The "color difference" can be obtained by using a commercially available colorimeter to perform colorimetry on two points (two regions) for measurement, and then comparing numerical values obtained based on the CIE1976 (L*a*b*) color space defined in JIS Z 8729 or the like. Specifically, letting ΔL* be the difference between the L* values of the two points for measurement, Δa* be the difference between the a* values, and Δb* be the difference between the b* values, the color difference ΔE*ab can be obtained by [(ΔL*)²+(Δa*)²+(Δb*)²]^{1/2}. In the present disclosure, it is assumed that "have mutually different colors" is satisfied when this color difference ΔE*ab is greater than or equal to 1.5, or desirably greater than or equal to 3.0. Since the value of ΔE*ab is greater than or equal to 1.5, it can be said that the colors are different to the extent of being detectable by a human with the unaided eye, and therefore the user can sufficiently recognize the cutout 45.

Further, cases where a predetermined region of the sheet member "has been given a color" include the case where the region is given a hue made up of a single color or a gradation, and the case where an image including text, a pattern, or the like is provided such that the color of the image portion is different from the color of the nonwoven fabric material. Also, when actually realizing the state "has been given a color", the sheet member may be subjected to printing or the like so as to have a different color from another sheet member, or the color of the sheet member itself (e. g., the color of a material such as the fibers configuring the sheet member) may be different from another sheet member.

In consideration of the fact that the nonwoven fabric configuring the diaper 1 normally has a white-based color, it is desirable that when giving the sheet member a color, a blue-based color is used in order to given a cool/refreshing feeling and a feeling of cleanliness. Further, in the case of using a chromatic colored sheet as the separate sheet, when one of the two points for measurement is the color of the chromatic colored sheet, and the other point for measurement is a color that is in a complimentary relationship with the color of the chromatic colored sheet, it is easier to recognize the difference between the colors of the two points.

FIG. 5B illustrates a state where the arrangement of the separate sheet member 50 is different from the case in FIG. 5A. In FIG. 5B, the upper end 50b in the vertical direction of the separate sheet member 50 is arranged lower than the upper end 40b in the vertical direction of the front waist portion 40. As a result, as illustrated by the solid black portion in FIG. 5B, the separate sheet member 50 is overlapped with the lower region of the edge portion 46, and is not overlapped with the upper region. In at least a part of this overlap region, the color of the separate sheet member 50 is different from the color of the nonwoven fabric that configures the back waist portion 30. Accordingly, the back waist portion 30 and at least a part of the region illustrated by the solid black portion of the edge portion 46 have mutually different colors separated at the outer edge of the cutout 45, and the edge portion 46 is more prominent, thus making it easier for the user to recognize the cutout 45. In this way, in the case where the color of even a part of the edge portion 46 is different, the cutout 45 can be recognized. It should be noted that by giving a different color to the widest range of the edge portion 46 as possible, the identifiability of that region increases (see FIG. 5A), thus making it possible to make the cutout 45 even easier to recognize.

When giving the separate sheet member 50 a color, it is possible to provide any region with any color or image or the like, by design. For this reason, by providing a design that makes the region overlapped with the edge portion 46 more prominent, the cutout 45 can be efficiently recognized easier, and the design of the diaper 1 can be enhanced.

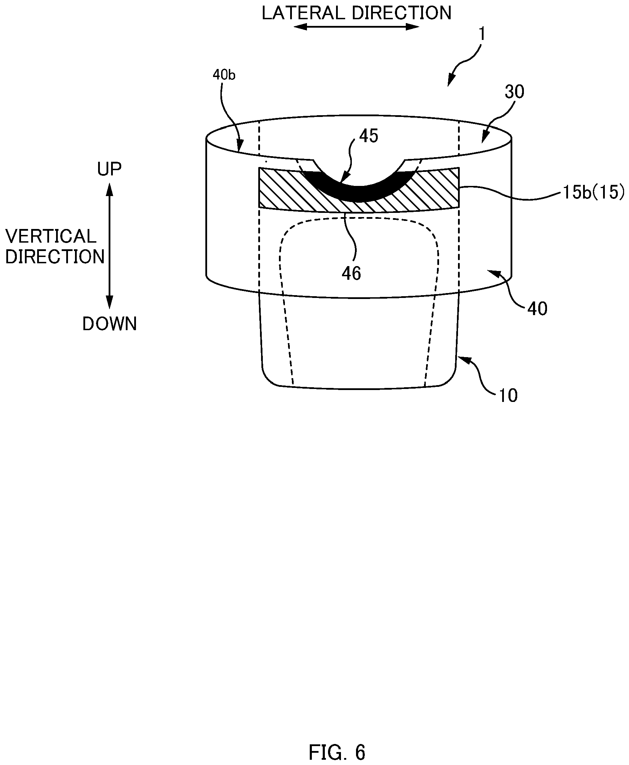

Further, the cutout 45 can be made easier to recognize by giving a color to a predetermined region of another sheet member other than the separate sheet member 50 as the sheet member overlaid in the waist portion. FIG. 6 is a diagram illustrating an example of the case where a portion on the front side of the back-sheet member 15 of the absorbent main body 10 has a different color. In FIG. 6, a color is given to the upper end portion region in the vertical direction on the front side (the region illustrated by the hatched portion in FIG. 6) of the leak-proof sheet 15a provided on the non-skin side of the absorbent main body 10. The front waist portion 40 and the leak-proof sheet 15a are arranged so as to be overlaid such that this region illustrated by hatching is overlapped with at least a part of the edge portion 46. As a result, in the region of the edge portion 46 illustrated by the solid black portion in FIG. 6, the color given to the leak-proof sheet 15a can be seen through the front waist portion 40. Accordingly, the back waist portion 30 and at least a part of the region illustrated by the solid black portion of the edge portion 46 have mutually different colors separated at the outer edge of the cutout 45, and the cutout 45 is easier to recognize.

Note that in the example in FIG. 6, the upper end portion in the vertical direction on the front side of the absorbent main body 10 (leak-proof sheet 15a) is located lower than the upper end 40b in the vertical direction of the front waist portion 40, but the upper end portion in the vertical direction on the front side of the leak-proof sheet 15a may be located at the same height as the upper end 40b in the vertical direction of the front waist portion 40. Further, in the above example, the case where a color is given to the leak-proof sheet 15a of the back sheet member 15 is described, but a color may be given to the exterior sheet 15b. In either case, by giving a color to the back sheet member 15, it is possible to make the cutout 45 easier to recognize even when the separate sheet member 50 or the like is not provided. Further, in the process for manufacturing the diaper 1, different colors can be given in a simple manner by performing printing on a portion of the back-sheet member 15, for example, and therefore the processing for manufacturing the diaper 1 does not become complex, and a rise in manufacturing cost can be restrained.

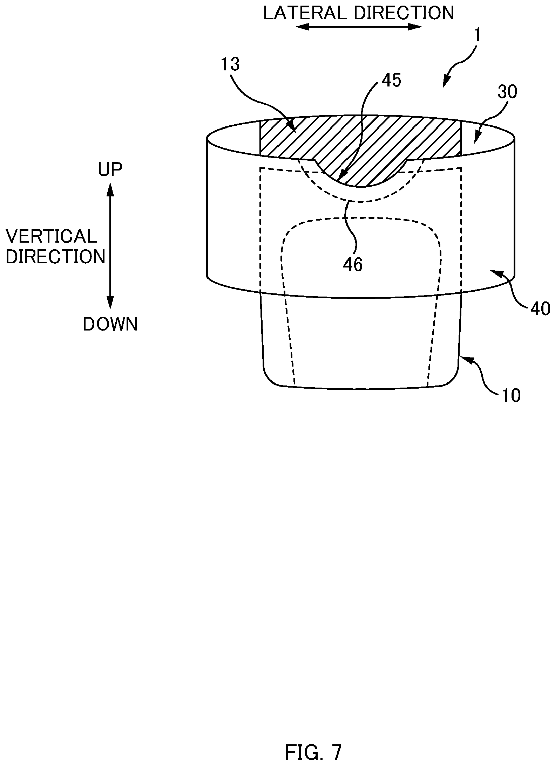

FIG. 7 is a diagram illustrating an example of the case where a portion on the back side of the top-sheet member 13 of the absorbent main body 10 has a different color. In FIG. 7, a color, a pattern, or the like has been given to the upper end portion region in the vertical direction (region illustrated by the hatched portion in FIG. 7) on the back side of the top-sheet member 13 that is provided most inward (on the skin side) in the absorbent main body 10. The back waist portion 30 and the top-sheet member 13 are arranged so as to be overlaid such that this region illustrated by hatching is overlapped with at least a part of the position of the back waist portion 30 that faces the cutout 45. As a result, in a view from the front side, the color given to the top-sheet member 13 can be seen through the cutout 45 as illustrated in FIG. 7. Accordingly, the back waist portion 30 and at least a part of the edge portion 46 have mutually different colors separated at the outer edge of the cutout 45, and the cutout 45 is easier to recognize. In this case as well, similarly to the case of giving a color to the back-sheet member 15, there is no need to provide another sheet member or the like, and manufacturing cost can be reduced.

In this way, by providing a sheet member on the waist portion 30 or 40 of the diaper 1, and giving a color to a predetermined region of the sheet member, different colors separated at the outer edge of the cutout 45 can be seen. Accordingly, the shape and position of the cutout 45 can be more easily recognized at a glance, and it is easier to distinguish between the front and the back of the diaper 1. Further, the color given to the sheet member, the region that is given a color, and the like can be changed freely, thus increasing the degree of freedom in design, and making it possible to give different colors such that the user can more easily recognize the cutout 45 according to differences in the type, shape, size, and the like of the diaper 1.

### ===Second Embodiment===

In a second embodiment, an example is described in which, in the diaper 1, the position and the shape of the cutout 45 are made easier to recognize by giving a color to the front waist portion 40 or the back waist portion 30. The basic configuration of the diaper 1 is substantially the same as in the first embodiment, and thus will not be described.

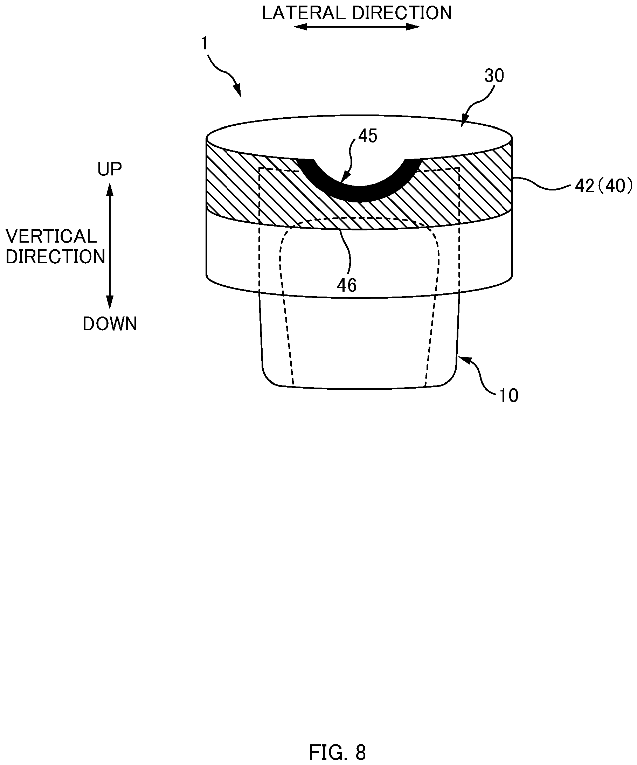

FIG. 8 is a diagram illustrating an example of the case where a partial region on the non-skin side of the front waist portion 40 has a different color. In FIG. 8, in the non-skin side sheet 42 of the front waist portion 40, a color is given to a region illustrated by the hatched portion on the upper side of the non-skin side surface. This region illustrated by hatching is overlapped with the edge portion 46 in the solid block portion in FIG. 8, and thus this overlapping portion appears to have a different color from the back waist portion 30. In other words, the back waist portion 30 and at least a part of the edge portion 46 have mutually different colors separated at the outer edge of the cutout 45, and the cutout 45 is easier to recognize.

Note that in the example in FIG. 8, a color is given to the non-skin side sheet 42 so as to be overlapped with the entire region of the edge portion 46, but it is not necessarily required that a color is given to the portion that overlaps the entire region of the edge portion 46, and it is sufficient that a color is given to a portion of the non-skin side sheet 42 that is overlapped with a portion of the edge portion 46. For example, as a portion of the surface design of the diaper 1, by providing a color or an image so as to overlap the edge portion 46, the design of the diaper 1 can be improved. Further, nonwoven fabric materials that have mutually different colors may be used as the nonwoven fabric that configures the non-skin side sheet 42 and the nonwoven fabric that configures the back waist portion 30. According to this configuration, it is possible to omit a step for printing or the like in the manufacturing process, and a diaper 1 with an easily recognizable cutout portion 45 can be manufactured more simply.

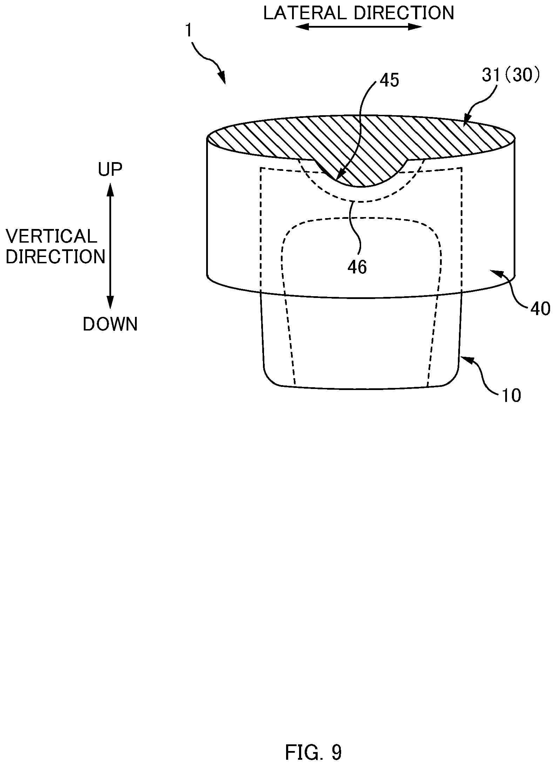

FIG. 9 is a diagram illustrating an example of the case where a partial region on the skin side of the back waist portion 30 has a different color. In FIG. 9, a color is given to a region illustrated by the hatched portion of the skin-side surface of the skin-side sheet 31 of the back waist portion 30. This region illustrated by the hatched portion is overlapped with at least a part of the position of the back waist portion 30 that faces the cutout 45. As a result, in a view from the front side, the color given to the skin-side sheet 31 can be seen through the cutout 45 as illustrated in FIG. 9. Accordingly, the back waist portion 30 and at least a part of the edge portion 46 have mutually different colors separated at the outer edge of the cutout 45, and recognition of the cutout 45 is facilitated.

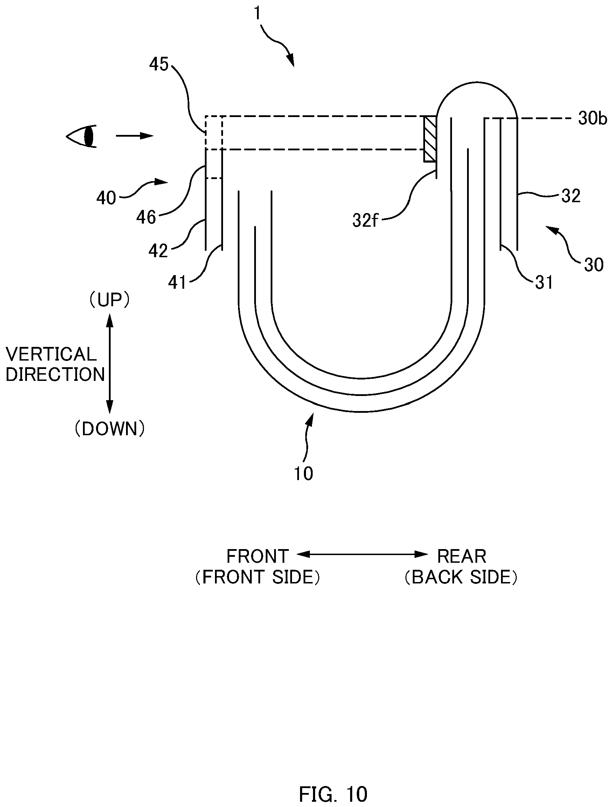

Also, the configuration of the non-skin side sheet 32 of the back waist portion 30 may be changed, and a color may be given to a predetermined region of this non-skin side sheet 32. FIG. 10 is a schematic cross-sectional view of the diaper 1 (pull-on shape) for illustrating an example of the case where the configuration of the non-skin side sheet 32 has been changed. In the example illustrated in FIG. 10, the length in the vertical direction of the non-skin side sheet 32 of the back waist portion 30 is longer than the length in the vertical direction of the skin-side sheet 31, and an upper end portion in the vertical direction of the non-skin side sheet 32 protrudes above the upper end 30b in the vertical direction of the back waist portion 30. A protruding portion 32f of the non-skin side sheet 32 is folded back on the skin side at the position of the upper end 30b in the vertical direction of the back waist portion 30, and is overlapped on and bonded to the absorbent main body 10. In this way, by folding back and layering the non-skin side sheet 32 in the region of the upper end 30b in the vertical direction of the back waist portion 30, the strength on the back side of the waist opening 1a is raised, and the wearer's skin (back side) is restrained from coming into direct contact with the absorbent main body 10 and the upper end of the skin-side sheet 31 at the waist opening 1a, which can provide more favorable texture at the waist opening 1a.

When the non-skin side sheet 32 is folded back and bonded, a color is given to the skin-side surface of the protruding portion 32f that is folded back. This folded-back protruding portion 32f is arranged at a position facing the cutout 45 in the back waist portion 30. For this reason, when viewing the diaper 1 from the front side, the color given to the folded-back protruding portion 32f can be viewed through the cutout 45, as illustrated by the hatched portion in FIG. 10. Accordingly, the back waist portion 30 and at least a part of the edge portion 46 have mutually different colors separated at the outer edge of the cutout 45, and the cutout 45 is easier to recognize.

### ===Third Embodiment===

In a third embodiment, an example is described in which, in the diaper 1, the position and the shape of the cutout 45 are made easier to recognize by giving a color to a portion of the elastic strings 43a and 43b provided in the front waist portion 40. The basic configuration of the diaper 1 is substantially the same as in the first embodiment, and thus will not be described.

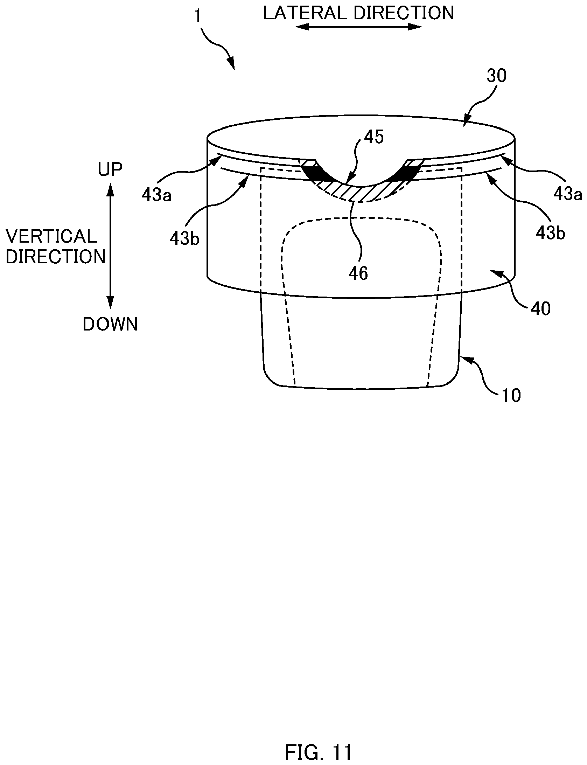

FIG. 11 is a diagram illustrating an example of the case where partial regions of elastic strings 43a and 43b have a different color. The elastic strings 43a and 43b are provided so as to intersect the cutout 45 in a central portion in the lateral direction. In other words, the elastic strings 43a and 43b are arranged so as to partially be overlapped with the edge portion 46. In FIG. 11, the elastic strings 43a and 43b and the edge portion 46 are overlapped in portions illustrated in solid black. In the third embodiment, a color is given to the elastic strings 43a and 43b in parts of the overlapping portions. Accordingly, the back waist portion 30 and at least a part of the edge portion 46 have mutually different colors separated at the outer edge of the cutout 45, and recognition of the cutout 45 is facilitated.

Also, in the third embodiment, in the case where elastic members (elastic strings) that have been given a color or the like in advance are used as the elastic strings 43a and 43b, there is no need for a printing step or the like in the process for manufacturing the diaper 1, thus making manufacturing simpler, and also making it possible to reduce manufacturing cost. Also, stretchable nonwoven fabric, stretchable urethane, or the like may be used instead of the elastic strings 43 as the elastic members provided in the front waist portion 40.

### ===Other embodiments===

Although embodiments of the present disclosure have been described above, the above embodiments are to facilitate understanding of the present disclosure and are not to interpret the present disclosure in a limiting manner. Also, the present disclosure can be modified and improved, and equivalents of the present disclosure are, needless to say, encompassed within the present disclosure. For example, variations such as the following are possible.

In embodiments of the present disclosure described above, a so-called three-piece type of diaper 1, which is configured with the three parts 10, 30, and 40, is described as an example of an absorbent article, but the absorbent article is not limited to this type. For example, the present disclosure is also applicable to a pull-on disposable diaper or the like that is configured with two parts: the absorbent main body 10; and an integrated exterior member in which the front waist portion 40 and the back waist portion 30 are integrally configured. In this case as well, a cutout is provided in the integrated exterior member at a location that is in the central portion in the lateral direction of the front waist portion and that is in the upper end portion in the vertical direction. Further, in a view from the front side, at least a partial region of the edge portion that includes the outer edge of the cutout in the front waist portion and at least a partial region of the back waist portion have mutually different colors separated at the outer edge of the cutout. Accordingly, the position and the shape of the cutout provided in the front waist portion are easier to recognize, and a mistake in the front-back direction of the diaper is restrained.

### List of Reference Numerals

1 diaper, 1a waist opening, 1b leg opening,
10 absorbent main body, 10a one end portion, 10b other end portion,
11 absorbent body, 11a back-side upper end, 11b front-side upper end,
12 recessed portion,
13 top-sheet member,
15 back-sheet member, 15a leak-proof sheet, 15b exterior sheet,
26 side flap, 26a LG elastic string,
28 top part, 29 LSG elastic string,
30 back waist portion, 30a back-side edge portion, 30b upper end,
31 skin-side sheet, 32 non-skin side sheet, 32f protruding portion,
33 back elastic string,
40 front waist portion, 40a front-side edge portion, 40b upper end, 40c two side portions,
41 skin-side sheet, 42 non-skin side sheet,
45 cutout, 45a lower end, 41b side end,
43 front elastic string, 43a, 43b elastic string,
46 edge portion,
50 separate sheet member, 50b upper end,
LG leg gather,
LSG leg side gather

## Claims

1. A pull-on disposable diaper (1) having a vertical direction and a lateral direction intersecting the vertical direction, the pull-on disposable diaper comprising:
an absorbent main body (10) provided with an absorbent body (11) that absorbs bodily wastes;
a back waist portion (30) located on one end side of the absorbent main body; and
a front waist portion (40) located on another end side of the absorbent main body,
the front waist portion (40) including a cutout (45), the cutout being located in an upper end portion (40b) of the front waist portion in the vertical direction and in a central portion of the front waist portion in the lateral direction, and
in a view from a front side, at least a partial region of an edge portion (46) including an outer edge of the cutout (45) in the front waist portion (40) and at least a partial region of the back waist portion (30) having mutually different colors separated at the outer edge of the cutout (45), wherein
a color difference between a color in at least a partial region of the edge portion (46) including the outer edge of the cutout (45) in the front waist portion (40) and a color in at least a partial region of the back waist portion (30) is greater than or equal to 1.5, the color difference being calculated based on an L*a*b* color coordinate system, measured as described in the description,
a sheet member (50) overlapped with at least a part of the edge portion (46) is overlaid in the front waist portion (40), and
in a view from the front side, an overlap region of the sheet member (50) and the edge portion (46) and at least a partial region of the back waist portion (30) have mutually different colors separated at the outer edge of the cutout (45).

2. A pull-on disposable diaper according to claim 1, wherein
the sheet member (50) is configured by a film or nonwoven fabric,
the sheet member (50) is a design sheet with a predetermined color or image provided on its surface and provided on the front waist portion (40), and
the design sheet has a region overlapped with the edge portion (46), and a color is given to at least a part of the region overlapped with the edge portion.

3. A pull-on disposable diaper according to claim 1, wherein
the absorbent main body (10) has a top sheet (13) that covers the absorbent body (11) from a skin side, and a back sheet (15) that covers the absorbent body from a non-skin side,
the sheet member is the back sheet (15), and
the back sheet (15) has a region overlapped with the edge portion (46), and a color is given to at least a part of the region overlapped with the edge portion.

4. A pull-on disposable diaper according to claim 1, wherein
a sheet member (50) is overlaid in the back waist portion (30), the sheet member being overlapped with at least a part of a position facing the cutout (45), and
in a view from a front side, the edge portion (46) and an overlap region of the position facing the cutout (45) and the sheet member (50) have mutually different colors separated at the outer edge of the cutout.

5. A pull-on disposable diaper according to claim 4, wherein
the absorbent main body (10) has a top sheet (13) that covers the absorbent body (11) from a skin side, and a back sheet (15) that covers the absorbent body from a non-skin side,
the sheet member is the top sheet (13), and
the back sheet (15) has a region where the sheet member and the position facing the cutout (45) are overlapped, and a color is given to at least a part of the region where the sheet member and the position facing the cutout are overlapped.

6. A pull-on disposable diaper according to any one of claims 1 to 5, wherein
a color is given to at least a partial region of the edge portion (46) on a non-skin side surface of the front waist portion (40).

7. A pull-on disposable diaper according to any one of claims 1 to 6, wherein
a skin-side surface of the back waist portion (30) has a position facing the cutout (45), and a color is given to at least a partial region of the position facing the cutout.

8. A pull-on disposable diaper according to any one of claims 1 to 7, wherein
the back waist portion (30) has a skin-side sheet (31) and a non-skin side sheet (32) overlaid in the thickness direction,
the non-skin side sheet has a protruding portion (32f) protruding upward from an upper end portion of the skin-side sheet (31), and the protruding portion is folded back on a skin side, and
a folded-back portion of the non-skin side sheet (32) has a position facing the cutout (45), and a color is given to at least a partial region of the position facing the cutout.

9. A pull-on disposable diaper according to any one of claims 1 to 8, wherein
the front waist portion (40) is provided with an elastic member (43a) that gives stretchability in the lateral direction, the elastic member being overlapped with at least a part of the edge portion (46), and
the elastic member (43a) has a region overlapped with the edge portion (46), and a color is given to at least a part of the region overlapped with the edge portion.

## Patentansprüche

1. Einwegschlupfwindel (1), der eine vertikale Richtung und eine laterale Richtung, die die vertikale Richtung schneidet, aufweist, wobei die Einwegschlupfwindel Folgendes umfasst:
einen absorbierenden Hauptkörper (10), der mit einem Saugkörper (11) bereitgestellt ist, der Körperausscheidungen absorbiert;
einen hinteren Taillenteil (30), der sich auf einer Endseite des absorbierenden Hauptkörpers befindet, und
einen vorderen Taillenteil (40), der sich auf einer anderen Endseite des absorbierenden Hauptkörpers befindet,
wobei der vordere Taillenteil (40) einen Ausschnitt (45) einschließt, wobei sich der Ausschnitt in einem oberen Endteil (40b) des vorderen Taillenteils in der vertikalen Richtung und in einem mittleren Teil des vorderen Taillenteils in der lateralen Richtung befindet, und
in einer Ansicht von einer Vorderseite mindestens ein Teilbereich eines Randteils (46), der einen äußeren Rand des Ausschnitts (45) in dem vorderen Taillenteil (40) einschließt, und mindestens ein Teilbereich des hinteren Taillenteils (30) voneinander verschiedene Farben aufweisen, die an dem äußeren Rand des Ausschnitts (45) getrennt sind, wobei
ein Farbunterschied zwischen einer Farbe in mindestens einem Teilbereich des Randteils (46), der einen äußeren Rand des Ausschnitts (45) in dem vorderen Taillenteil (40) einschließt, und einer Farbe in mindestens einem Teilbereich des hinteren Taillenteils (30) größer oder gleich 1,5 ist, wobei der Farbunterschied basierend auf einem L*a*b*-Farbkoordinatensystem berechnet wird, wie in der Beschreibung beschrieben gemessen wird,
ein Lagenelement (50), das mit mindestens einem Teil des Randteils (46) überlappt ist, in dem vorderen Taillenteil (40) überlagert ist und
in einer Ansicht von der Vorderseite ein Überlappungsbereich des Lagenelements (50) und des Randteils (46) und mindestens ein Teilbereich des hinteren Taillenteils (30) voneinander verschiedene Farben aufweisen, die an dem äußeren Rand des Ausschnitts (45) getrennt sind.

2. Einwegschlupfwindel nach Anspruch 1, wobei
das Lagenelement (50) durch einen Film oder Vliesstoff konfiguriert ist,
das Lagenelement (50) eine Design-Lage mit einer vorgegebenen Farbe oder Abbildung ist, die auf dessen Oberfläche bereitgestellt sind und auf dem vorderen Taillenteil (40) bereitgestellt sind, und
die Design-Lage einen Bereich aufweist, der mit dem Randteil (46) überlappt ist, und mindestens einem Teil des Bereichs, der mit dem Randteil überlappt ist, eine Farbe gegeben ist.

3. Einwegschlupfwindel nach Anspruch 1, wobei
der absorbierende Hauptkörper (10) Folgendes aufweist: eine obere Lage (13), die den Saugkörper (11) von einer Hautseite bedeckt, und eine hintere Lage (15), die den Saugkörper von einer Nicht-Hautseite bedeckt,
das Lagenelement die hintere Lage (15) ist und
die hintere Lage (15) einen Bereich aufweist, der mit dem Randteil (46) überlappt ist, und mindestens einem Teil des Bereichs, der mit dem Randteil überlappt ist, eine Farbe gegeben ist.

4. Einwegschlupfwindel nach Anspruch 1, wobei
ein Lagenelement (50) in dem hinteren Taillenteil (30) überlagert ist, wobei das Lagenelement mit mindestens einem Teil einer Position, die dem Ausschnitt (45) zugewandt ist, überlappt ist, und
in einer Ansicht von einer Vorderseite der Randteil (46) und ein Überlappungsbereich der Position, die dem Ausschnitt (45) zugewandt ist, und des Lagenelements (50) voneinander verschiedene Farben aufweisen, die an dem äußeren Rand des Ausschnitts getrennt sind.

5. Einwegschlupfwindel nach Anspruch 4, wobei
der absorbierende Hauptkörper (10) Folgendes aufweist: eine obere Lage (13), die den Saugkörper (11) von einer Hautseite bedeckt, und eine hintere Lage (15), die den Saugkörper von einer Nicht-Hautseite bedeckt,
das Lagenelement die obere Lage (13) ist und
die hintere Lage (15) einen Bereich aufweist, wo das Lagenelement und die Position, die dem Ausschnitt (45) zugewandt ist, überlappt sind, und mindestens einem Teil des Bereichs, wo das Lagenelement und die Position, die dem Ausschnitt zugewandt ist, überlappt sind, eine Farbe gegeben ist.

6. Einwegschlupfwindel nach einem der Ansprüche 1 bis 5, wobei
mindestens einem Teilbereich des Randteils (46) auf einer Nicht-Hautseitenoberfläche des vorderen Taillenteils (40) eine Farbe gegeben ist.

7. Einwegschlupfwindel nach einem der Ansprüche 1 bis 6, wobei
eine Hautseitenoberfläche des hinteren Taillenteils (30) eine Position aufweist, die dem Ausschnitt (45) zugewandt ist, und mindestens einem Teilbereich der Position, die dem Ausschnitt zugewandt ist, eine Farbe gegeben ist.

8. Einwegschlupfwindel nach einem der Ansprüche 1 bis 7, wobei
der hintere Taillenteil (30) eine Hautseitenlage (31) und eine Nicht-Hautseitenlage (32) aufweist, die in der Dickenrichtung überlagert sind,
die Nicht-Hautseitenlage einen vorstehenden Teil (32f) aufweist, der nach oben von einem oberen Endteil der Hautseitenlage (31) vorsteht, und der vorstehende Teil auf einer Hautseite zurückgefaltet ist und
ein zurückgefalteter Teil der Nicht-Hautseitenlage (32) eine Position aufweist, die dem Ausschnitt (45) zugewandt ist, und mindestens einem Teilbereich der Position, die dem Ausschnitt zugewandt ist, eine Farbe gegeben ist.

9. Einwegschlupfwindel nach einem der Ansprüche 1 bis 8, wobei
der vordere Taillenteil (40) mit einem elastischen Element (43a) bereitgestellt ist, das in der lateralen Richtung eine Dehnbarkeit verleiht, wobei das elastische Element mit mindestens einem Teil des Randteils (46) überlappt ist, und
das elastische Element (43a) einen Bereich aufweist, der mit dem Randteil (46) überlappt ist, und mindestens einem Teil des Bereichs, der mit dem Randteil überlappt ist, eine Farbe gegeben ist.

## Revendications

1. Couche jetable de type culotte (1) ayant une direction allant dans le sens vertical et une direction allant dans le sens latéral qui croise la direction allant dans le sens vertical, la couche jetable de type culotte comportant :
un corps principal absorbant (10) comportant un corps absorbant (11) qui absorbe les déjections corporelles ;
une partie arrière au niveau de la taille (30) située sur un côté d'extrémité du corps principal absorbant ; et
une partie avant au niveau de la taille (40) située sur un autre côté d'extrémité du corps principal absorbant,
la partie avant au niveau de la taille (40) comprenant une découpe (45), la découpe étant située dans une partie d'extrémité supérieure (40b) de la partie avant au niveau de la taille dans la direction allant dans le sens vertical et dans une partie centrale de la partie avant au niveau de la taille dans la direction allant dans le sens latéral, et
dans une vue depuis un côté avant, au moins une région partielle d'une partie de bord (46) comprenant un bord extérieur de la découpe (45) dans la partie avant au niveau de la taille (40) et au moins une région partielle de la partie arrière au niveau de la taille (30) ayant mutuellement des couleurs différentes séparées au niveau du bord extérieur de la découpe (45), dans laquelle
une différence de couleur entre une couleur dans au moins une région partielle de la partie de bord (46) comprenant le bord extérieur de la découpe (45) dans la partie avant au niveau de la taille (40) et une couleur dans au moins une région partielle de la partie arrière au niveau de la taille (30) est supérieure ou égale à 1,5, la différence de couleur étant calculée en se basant sur un système de coordonnées de couleur L*a*b*, que l'on mesure tel qu'il est décrit dans la description,
un élément formant feuille (50) chevauché par au moins une partie de la partie de bord (46) est superposé dans la partie avant au niveau de la taille (40), et
dans une vue depuis le côté avant, une région de chevauchement de l'élément formant feuille (50) et de la partie de bord (46) et au moins une région partielle de la partie arrière au niveau de la taille (30) ont mutuellement des couleurs différentes séparées au niveau du bord extérieur de la découpe (45) .

2. Couche jetable de type culotte selon la revendication 1, dans laquelle
l'élément formant feuille (50) est configuré par un film ou un tissu non tissé,
l'élément formant feuille (50) est une feuille de conception avec une image ou une couleur prédéterminée mise en oeuvre sur sa surface et mise en oeuvre sur la partie avant au niveau de la taille (40), et
la feuille de conception a une région chevauchée par la partie de bord (46), et une couleur est donnée à au moins une partie de la région chevauchée par la partie de bord.

3. Couche jetable de type culotte selon la revendication 1, dans laquelle
le corps principal absorbant (10) a une feuille de dessus (13) qui recouvre le corps absorbant (11) depuis un côté orienté vers la peau, et une feuille de support (15) qui recouvre le corps absorbant depuis un côté non orienté vers la peau,
l'élément formant feuille est la feuille de support (15), et
la feuille de support (15) a une région chevauchée par la partie de bord (46), et une couleur est donnée à au moins une partie de la région chevauchée par la partie de bord.

4. Couche jetable de type culotte selon la revendication 1, dans laquelle
un élément formant feuille (50) est superposé dans la partie arrière au niveau de la taille (30), l'élément formant feuille étant chevauché par au moins une partie d'une position orientée vers la découpe (45), et
dans une vue depuis un côté avant, la partie de bord (46) et une région de chevauchement de la position orientée vers la découpe (45) et l'élément formant feuille (50) ont mutuellement des couleurs différentes séparées au niveau du bord extérieur de la découpe.

5. Couche jetable de type culotte selon la revendication 4, dans laquelle
le corps principal absorbant (10) a une feuille de dessus (13) qui recouvre le corps absorbant (11) par rapport à un côté orienté vers la peau, et une feuille de support (15) qui recouvre le corps absorbant par rapport à un côté non orienté vers la peau,
l'élément formant feuille est une feuille de dessus (13), et
la feuille de support (15) a une région où l'élément formant feuille et la position orientée vers la découpe (45) se chevauchent l'une l'autre, et une couleur est donnée à au moins une partie de la région où l'élément formant feuille et la position orientée vers la découpe se chevauchent l'une l'autre.

6. Couche jetable de type culotte selon l'une quelconque des revendications 1 à 5, dans laquelle
une couleur est donnée à au moins une région partielle de la partie de bord (46) sur une surface de côté non orienté vers la peau de la partie avant au niveau de la taille (40).

7. Couche jetable de type culotte selon l'une quelconque des revendications 1 à 6, dans laquelle
une surface de côté orienté vers la peau de la partie arrière au niveau de la taille (30) a une position orientée vers la découpe (45), et une couleur est donnée à au moins une région partielle de la position orientée vers la découpe.

8. Couche jetable de type culotte selon l'une quelconque des revendications 1 à 7, dans laquelle
la partie arrière au niveau de la taille (30) a une feuille côté orienté vers la peau (31) et une feuille côté non orienté vers la peau (32) superposées dans la direction allant dans le sens de l'épaisseur,
la feuille côté non orienté vers la peau a une partie faisant saillie (32f) qui fait saillie vers le haut depuis une partie d'extrémité supérieure de la feuille côté orienté vers la peau (31), et la partie faisant saillie est repliée sur un côté orienté vers la peau, et
une partie repliée de la feuille côté non orienté vers la peau (32) a une position orientée vers la découpe (45), et une couleur est donnée à au moins une région partielle de la position orientée vers la découpe.

9. Couche jetable de type culotte selon l'une quelconque des revendications 1 à 8, dans laquelle
la partie avant au niveau de la taille (40) comporte un élément élastique (43a) qui donne de l'extensibilité dans la direction allant dans le sens latéral, l'élément élastique étant chevauché par au moins une partie de la partie de bord (46), et
l'élément élastique (43a) a une région chevauchée par la partie de bord (46), et une couleur est donnée à au moins une partie de la région chevauchée par la partie de bord.
